# EUROPÄISCHE PATENTSCHRIFT

(11) **EP 2 147 314 B1**
(45) Veröffentlichungstag und Bekanntmachung des Hinweises auf die Patenterteilung: **15.04.2015**
(21) Anmeldenummer: 08714306.1
(22) Anmeldetag: 27.03.2008
(51) Int. Cl.: G01N 21/17, G01N 33/543, B82Y 15/00, B82Y 30/00, G01N 21/25

(54) **OPTISCHES MESSVERFAHREN ZUR MOLEKULAREN DETEKTION ANHAND VON RELAXATIONSMESSUNGEN AN OPTISCH ANISOTROPEN NANOPARTIKELN**
OPTICAL MEASUREMENT METHOD FOR MOLECULAR DETECTION USING RELAXATION MEASUREMENTS AT OPTICALLY ANISOTROPIC NANOPARTICLES
PROCÉDÉ OPTIQUE DE MESURE POUR LA DÉTECTION MOLÉCULAIRE AU MOYEN DE MESURES DE RELAXATION RELATIVES À DES NANOPARTICULES OPTIQUEMENT ANISOTROPES

(30) Priorität: 11.04.2007 AT 5602007
(43) Veröffentlichungstag der Anmeldung: 27.01.2010
(73) Patentinhaber: AIT Austrian Institute of Technology GmbH, 1220 Wien (AT); tecnet equity NÖ Technologiebeteiligungs-Invest GmbH, 3100 St. Pölten (AT)
(72) Erfinder: BRÜCKL, Hubert, A-2344 Maria Enzersdorf (AT); SCHOTTER, Jörg, A-1050 Wien (AT); BETHGE, Ole, A-1070 Wien (AT)
(74) Vertreter: Wildhack & Jellinek
(86) Internationale Anmeldenummer: PCT/AT2008/000110
(87) Internationale Veröffentlichungsnummer: WO 2008/124853

(56) Entgegenhaltungen:
- WO-A-01/11360
- GLOCKL G ET AL: "Development of a liquid phase immunoassay by time-dependent measurements of the transient magneto-optical birefringence using functionalized magnetic nanoparticles" JOURNAL OF MAGNETISM AND MAGNETIC MATERIALS, ELSEVIER SCIENCE PUBLISHERS, AMSTERDAM, NL, Bd. 289, 1. März 2005 (2005-03-01), Seiten 480-483, XP004791182 ISSN: 0304-8853
- Bianca M. I. Van Der Zande ET AL: "Alignment of Rod-Shaped Gold Particles by Electric Fields", The Journal of Physical Chemistry B, vol. 103, no. 28, 1 July 1999 (1999-07-01) , pages 5754-5760, XP055107753, ISSN: 1520-6106, DOI: 10.1021/jp984737a

## Beschreibung

### Technisches Gebiet

Die Erfindung betrifft eine neue Nachweismethode bei einem molekularen Detektionsverfahren, bei welchem die nachzuweisenden Zielmoleküle spezifisch an die Oberfläche von in einer geeigneten Lösung suspendierten und entsprechend funktionalisierten Nanopartikeln anbinden. Durch die Anbindung vergrößern sich die hydrodynamischen Durchmesser der Partikel. Wird die Ausrichtung der Partikel nun durch einen externen Stimulus verändert, so führt die Vergrößerung der hydrodynamischen Durchmesser zu einem geänderten Brown'schen Relaxationsverhalten der Nanopartikel. Die Messung dieses Relaxationsverhaltens gestattet somit die Quantifizierung der Konzentration der in der Lösung befindlichen Zielmoleküle.

### Technische Aufgabe und Erfindung

Die Erfindung betrifft ein Verfahren zur molekularen Detektion gemäß dem **Oberbegriff** des **Anspruchs** 1, welches die im **Kennzeichen** dieses Anspruchs genannten **Merkmale** aufweist.

Kern der vorliegenden Erfindung ist also eine Art des Nachweises der Partikelrelaxation durch Nutzung von anisotropen optischen Eigenschaften der verwendeten Nanopartikel. Verändert sich die räumliche Ausrichtung der Partikel, so führt deren optische Anisotropie in einem geeigneten optischen Messaufbau zu einer entsprechenden optisch nachweisbaren Signalveränderung, welche die Bestimmung der momentanen Ausrichtung der Partikel absolut oder relativ zu einem externen Stimulus bzw. Feld gestattet. Mögliche Messgrößen sind hierbei beispielsweise, aber nicht ausschließlich, das Spektrum und/oder die Polarisation des von den Partikeln gestreuten, emittierten oder ausgelöschten Lichtes.

Die **Patentansprüche** 2 bis 8 betreffen verschiedene bevorzugte Ausführungsformen der vorliegenden Erfindung und Anspruch 9 betrifft eine im Rahmen der Erfindung vorteilhafte Mess-Einrichtung zur Durchführung des Verfahrens.

### Stand der Technik

Die Messung des Relaxationsverhaltens zur Bestimmung der Molekülbelegung von Nanopartikeln wurde erstmals von Weitschies at al. in der WO96/23227 A1 veröffentlicht, wo ferromagnetische oder ferrimagnetische Nanopartikel beschrieben werden, deren magnetische Momente in einem externen Magnetfeld ausgerichtet sind. Die Partikel sind hierbei so beschaffen, dass die Néel'sche Relaxationszeit der Partikelmagnetisierung größer ist als die Brown'sche Relaxationszeit, wobei letztere abhängig ist vom hydrodynamischen Durchmesser der Partikel, welcher sich bei einer Anlagerung von Molekülen vergrößert. Nachdem z.B. ein die Partikel ausrichtendes Magnetfeld ausgeschaltet wurde, wird der zeitliche Verlauf der Relaxation des magnetischen Moments des Partikelensembles mit Hilfe eines geeigneten Sensors aufgenommen, wodurch ein Rückschluss auf die durchschnittliche Molekülbelegung des Partikelensembles möglich ist. Als Sensoren können hierbei SQUID's, Induktionsspulen, Flux Gates oder magnetoresistive Elemente eingesetzt werden. Die Partikel befinden sich entweder frei in Lösung oder sind mittels bzw. über Fängermoleküle (h) spezifisch an Oberflächen gebunden.

Ein ähnliches Verfahren beschreiben Minchole et al. in der WO03/019188 A1, welches dasselbe Verfahrensprinzip betrifft, jedoch ein alternatives Messverfahren vorstellt, welches auf der Aufnahme eines magnetischen AC-Suszeptibilitätsspektrums des Partikelensembles beruht, woraus ebenfalls die mittlere Relaxationszeit der magnetischen Partikel bestimmt werden kann.

Allen bislang veröffentlichen Anmeldungs- und Patentschriften und Publikationen betreffend die Moleküldetektion mittels Relaxationsmessungen mit Nanopartikeln ist jedoch gemein, dass bisher ausschließlich rein magnetische Partikel und auf Magnetismus basierende Verfahren angewendet werden. Insbesondere in Bezug auf die Messung der Relaxation jedoch haben "magnetische" Messverfahren den Nachteil, dass das magnetische Moment des verwendeten Partikelensembles sehr klein ist und die magnetischen Streufelder mit der Entfernung vom Partikel sehr schnell abfallen, wodurch entweder hochsensitive, jedoch aufwändige Sensoren, wie SQUID's notwendig sind, oder aber die Sensoren räumlich sehr dicht bei den Partikeln platziert werden müssen, was die praktische Verwendbarkeit des Gesamtsystems sowie die Reproduzierbarkeit der Messergebnisse wesentlich einschränkt.

Optische Messverfahren bieten dagegen - wie gefunden wurde - den Vorteil exzellenter Sensitivität bei gleichzeitig hoher apparativer Toleranz. Insbesondere die Messgröße der Extinktion bzw. Auslöschung ist weitgehend abstandsunabhängig und erlaubt somit den Aufbau eines flexiblen Geräts mit hohem Probendurchsatz und guter Reproduzierbarkeit sowie Sensitivität der Messergebnisse.

Optische Messmethoden zur Relaxation von doppelbrechenden Nanopartikeln sind aus der WO 01/11360 A2 sowie aus G. Glöckl et al, "Development of a liquid phase immunoassay by time-dependent measurements of the transient magneto-optical birefringence using functionalized magnetic nanoparticles", Journal of Magnetism and Magnetic Materials 289 (2005) 480-483 bekannt.

In der klassischen Biotechnologie finden eine Vielzahl optisch aktiver Elemente Verwendung. Die Palette reicht dabei von organischen Fluoreszenzfarbstoffen über halbleitende Nanokristalle, auch Quantum Dots genannt, z.B. CdSe oder ZnS-Nanopartikel bis zu Nanopartikeln aus Edelmetallen wie Au oder Ag, in welchen durch einfallendes Licht Plasmonenresonanzen angeregt werden können, wodurch ein sehr charakteristisches Streu- und Auslöschungsspektrum entsteht. Bei den meisten Anwendungen werden diese optischen Elemente ausschließlich als Label verwendet, um die Präsenz bestimmter spezifisch markierter Moleküle nachzuweisen. Bei diesen Anwendungen müssen die Molekül-Label-Komplexe zumeist spezifisch an Oberflächen gebunden werden, damit ungebundene Label in nachfolgenden Waschschritten entfernt werden können und diese dann das Analyseergebnis nicht verfälschen.

Allerdings ist die Bindungseffektivität von Molekülen an Oberflächen verglichen mit Prozessen in der freien Lösung herabgesetzt, und die Analysezeit ist aufgrund der erforderlichen Diffusion der nachzuweisenden Moleküle zu der funktionalisierten Oberfläche deutlich höher als bei volumetrischen Reaktionen. Zudem sind die erforderlichen Oberflächenfunktionalisierungen für eine Vielzahl von Applikationen, bei welchen lediglich die schnelle Bestimmung der Konzentration weniger Zielmolekül-Arten in einem bestimmten Lösungsvolumen von Interesse ist, zu aufwändig.

Um allerdings bei volumetrischen Methoden zwischen gebundenen und ungebundenen Labeln zu unterscheiden, werden Verfahren benötigt, bei welchen die Label selbst zumindest eine messbare Eigenschaft verändern, wenn Zielmoleküle spezifisch an sie anbinden.

Im Bereich der optischen DNA-Detektion können hierzu beispielsweise die so genannten molecular beacons verwendet werden, bei welchen der FRET-Effekt (fluorescent resonant energy transfer) im ungebundenen Zustand des Labels eine Fluoreszenz-Emission unterdrückt. Erst, wenn die Zielsequenz an das Label andockt, öffnet sich der molecular beacon und Donor und Akzeptor werden voneinander räumlich getrennt, so dass eine Fluoreszenz-Emission des Labels entsteht. Allerdings ist der Einsatz der molecular beacons auf den Nachweis von relativ kurzen DNA- und RNA-Strängen begrenzt, d.h. beispielsweise Proteine können auf diese Weise nicht nachgewiesen werden.

Ein weiteres Beispiel für aktive Label in der Biotechnologie sind Au- oder Ag-Nanopartikel, bei welchen sich aufgrund einer Veränderung der Plasmonenresonanz-Bedingung die Absorptions- und Emissions-Spektren verschieben, wenn Moleküle an die Partikeloberfläche andocken. Allerdings ist diese Verschiebung recht gering, weshalb die Nachweisgrenze für Proteine mit diesem System typischerweise auf den unteren µM-Bereich begrenzt ist.

Eine weitere Variante aktiver Label ist in der Patentanmeldung US 2006/0008924 A1 (Anker et al.) beschrieben. Hierbei werden halbseitig passivierte magnetische Nanopartikel vorgestellt, bei welchen die Fluoreszenz-markierten Zielmoleküle nur an einer der beiden Halbkugeloberflächen der Label spezifisch anbinden können. Werden die Label anschließend durch ein magnetisches Wechselfeld in Rotation versetzt und wird die Fluoreszenz nur in einem begrenzten Raumwinkel beobachtet, so blinken die spezifisch an die Label gebundenen Floureszenz-markierten Zielmoleküle in der Rotationsfrequenz auf und können so von den ungebundenen Fluoreszenz-Farbstoffen sowie weiteren Hintergrundsignalen unterschieden werden. Allerdings hat diese Nachweismethode keinerlei Bezug zu den der vorliegenden Erfindung zugrunde liegenden optischen Relaxationsmessungen.

### Beschreibung des Erfindungsgegenstandes

Kern der vorliegenden Erfindung ist der optische Nachweis der Relaxation von Nanopartikeln durch Nutzung von optisch anisotropen Eigenschaften der verwendeten Partikel. Erfindungsgemäß handelt es sich bei den optisch anisotropen Eigenschaften um Plasmonenresonanzen. Mögliche Messgrößen sind hierbei beispielsweise, aber nicht ausschließlich, das Spektrum oder die Polarisation des von den Partikeln gestreuten, emittierten oder ausgelöschten Lichtes. Die Partikel sind überdies so beschaffen, dass ihre räumliche Ausrichtung in der Lösung durch einen externen Stimulus, insbesondere durch ein externes Feld, verändert werden kann, wie beispielsweise durch ein elektrisches oder magnetisches Feld.

Die Erfindung gestattet es somit, mittels optischer Verfahren die räumliche Ausrichtung der Partikel relativ zum Stimulus bzw. Feld zu bestimmen, was eine Messung der Partikel-Relaxation erlaubt. Werden an die Partikeloberfläche zudem bestimmte Zielmoleküle spezifisch angebunden, so erlaubt das gemessene Relaxationsverhalten eine Quantifizierung der Konzentration der Zielmoleküle in der Lösung. Das für das Verfahren eingesetzte Messsystem umfasst somit folgende Komponenten:

### 1. Externer Stimulus bzw. externes Feld:

Um die Nanopartikel in einer definierten Raumrichtung auszurichten, muss ein externer Stimulus ein Drehmoment auf die Partikel ausüben können. Dieser Stimulus kann insbesondere ein unidirektionales elektrisches oder magnetisches Feld sein.

Beispielsweise ist bekannt, dass längliche Nanopartikel aus Edelmetallen wie Au und Ag direkt durch das Anlegen von elektrischen Feldern orientiert werden können. Allerdings sind die erforderlichen elektrischen Feldstärken für das Erreichen eines ausreichenden Ausrichtungsgrades für praktische Anwendungen recht hoch, siehe z.B. van der Zande et al., J. Phys. Chem. B 103 (1999) 5754. Zudem werden durch das Anlegen eines starken elektrischen Feldes auch die Pufferlösung sowie die Zielmoleküle elektrisch polarisiert, was zu störenden Einflüssen führen kann.

Magnetische Felder dagegen haben den Vorteil, dass sie in biologischen Systemen kaum störend wirken. Um allerdings magnetische Felder für das Ausrichten der Nanopartikel verwenden zu können, müssen dieselben ein magnetisches Moment besitzen, dessen Néel'sche Relaxationszeit deutlich über der zu messenden Brown'schen Relaxationszeit liegt. Für den Fall, dass für die optischen Anisotropiemessungen nicht ohnehin magnetische Partikel verwendet werden, können nichtmagnetische Nanopartikel mit Hilfe eines geeigneten Komposit-Aufbaus mit magnetischen Komponenten versehen werden, um auch solche an sich nicht magnetischen durch magnetische Felder ausrichten zu können. Beispiele für einen solchen Komposit-Aufbau sind etwa Kern-Hülle-Strukturen oder längliche Partikel mit magnetischen Materialen an den Polen.

Zur Messung des Relaxationsverhaltens der Partikel wird der externe Stimulus in seiner Amplitude und/oder Richtung verändert, und die Relaxation der Partikel hin zu der durch den neue Stimulus gegebenen Gleichgewichtsposition wird zeit-, frequenz- und/oder phasenabhängig aufgezeichnet. Hierbei sind sowohl einzelne als auch periodische Veränderungen des Stimulus bzw. Feldes möglich.

Eine Einzelmessung kann beispielsweise aus dem Abschalten eines zuvor räumlich und zeitlich konstanten Stimulus bestehen, woraufhin sich die optischen Anisotropierichtungen des Ensembles der Nanopartikel durch die Brown'sche Relaxation im Lauf der Zeit wieder stochastisch verteilen. Damit verbunden ist eine zeitliche Abnahme des gemessenen optischen Anisotropiesignals des Ensembles der Nanopartikel, und aus der Abklingzeit kann auf die mittlere Beladung der Nanopartikel mit Zielmolekülen geschlossen werden.

Ein weiteres mögliches Beispiel für eine periodische Veränderung des Stimulus ist eine kontinuierliche räumliche Drehung bei konstanter Amplitude. In diesem Fall besteht das Messsignal aus der bei einer geeigneten Rotationsfrequenz aufgenommenen Phasenverschiebung zwischen der Richtung des Stimulus und der gemessenen mittleren Ausrichtung der optischen Anisotropieachsen des Nanopartikel-Ensembles, welche mit der mittleren Beladung der Nanopartikel mit Zielmolekülen zunimmt.

### 2. Messzelle:

Die Messzelle enthält in einer geeigneten Trägerflüssigkeit die optisch anisotropen Nanopartikel und die zu analysierende Substanz, welche neben den Zielmolekülen auch unspezifische Zusatzsubstanzen enthalten kann. Als Zielmoleküle kommen sowohl biologische Substanzen, wie z.B. Viren, Bakterien, Nukleinsäuresequenzen, Antikörper, Anigene oder beliebige andere Proteine, als auch gelöste anorganische oder organische chemische Substanzen, wie z.B. Polymere, in Betracht.

Die Trägerflüssigkeit ist hierbei mindestens in dem Wellenlängenbereich, welcher zum Vermessen der optischen Anisotropie der Nanopartikel verwendet wird, optisch transparent. Die Messzelle ist in diesem Wellenlängenbereich ebenfalls optisch transparent oder verfügt über Fenster, durch welche Licht in die Lösung eingestrahlt und ausgekoppelt werden kann.

### 3. Optisch anisotrope Nanopartikel:

Die Nanopartikel sind in all ihren Eigenschaften möglichst monodispers und besitzen mindestens eine optisch anisotrope Eigenschaft, welche die optische Erfassung der räumlichen Ausrichtung der Partikel in der Lösung gestattet. Zusätzlich kann durch einen externen Stimulus, also insbesondere ein elektrisches oder magnetisches Feld, ein Drehmoment auf die Partikel ausgeübt werden, um sie in einer bestimmten Raumrichtung auszurichten.

Die Partikel sind stabil in einer geeigneten Trägerflüssigkeit suspendiert, welche die nachzuweisenden Zielmoleküle enthält. Des Weiteren ist die Oberfläche der Partikel zumindest an einer Stelle so beschaffen, dass die Zielmoleküle spezifisch daran bzw. dort anbinden können.

Die Form der Partikel ist präferenziell länglich, um die Anforderungen hinsichtlich der optischen Anistropie und der Ausrichtbarkeit zu erfüllen. Jedoch sind auch abweichende Partikelformen nicht ausgeschlossen, wie z.B. sphärische Formen, wobei in diesem Fall die erforderliche optische Anisotropie und die Ausrichtbarkeit aus dem inneren Aufbau der Partikel folgen, z.B. durch die kristalline Anisotropie des Materials und/oder durch eine geeignete Komposit-Struktur mehrerer Materialien. Ein gut geeignetes Templat für längliche Komposit-Nanopartikel sind beispielsweise Carbon Nanotubes, welche mit geeigneten Materialien gefüllt und/oder umhüllt werden können, siehe hierzu Monthioux et al., Journal of Materials Research 21 (2006) 2774. Des Weiteren können auch spezifisch an den Nanotube-Enden bestimmte Materialien angelagert werden, siehe Jiang et al., Journal of Electroceramics 17 (2006) 87.

Die Größe der Partikel ist zum einen durch die Forderung begrenzt, dass die Partikel in der verwendeten Trägerflüssigkeit stabil gehalten sein müssen, d.h. sie dürfen nicht agglomerieren oder sedimentieren. Zum anderen muss eine Anbindung der Zielmoleküle zu einer messbaren Erhöhung der Brown'schen Relaxationszeit führen, d.h. die maximale Partikelgröße hängt von der Größe der nachzuweisenden Zielmoleküle ab. Aus diesen beiden Gründen sind nur Partikel praktisch sinnvoll verwendbar, deren Größe in jeder Dimension im Bereich von 1 bis 1000 Nanometern liegt.

Im Folgenden werden einige Beispiele für optisch anisotrope Nanopartikel aufgeführt:

### i) Metallische Nanopartikel mit spektraler Anisotropie

Diese Klasse von Partikel beinhaltet alle Arten von Nanopartikeln, bei welchen die spektrale Verteilung des von den Partikeln gestreuten, emittierten oder ausgelöschten Lichtes von der räumlichen Ausrichtung der optischen Anisotropieachsen der Partikel relativ zu einer Vorzugsrichtung abhängt. Diese Vorzugsrichtung kann hierbei durch Polarisatoren erzeugt werden, welche das einfallende und/oder das zu analysierende Licht polarisieren.

Eine mögliche Realisierungen solcher Partikel sind beispielsweise halbleitende Quantum Dots, Rods oder Wires, welche eine spektral anistrope Lumineszenz aufweisen. So werden etwa von Shan et al. CdSe Nanowires beschrieben, deren Emissionsspektrum aus zwei Bändern besteht, wobei deren relative Intensitäts-Anteile davon abhängen, ob das emittierte Licht parallel oder senkrecht zu den langen Achsen der Nanowires betrachtet wird, siehe Shan et al., Physical Review B 74 (2006) 153402.

Ein ähnliches Verhalten kann aber auch mit anderen Stoffen wie organischen Fluoreszenzfarbstoffen erreicht werden, sofern deren Spektrum über anisotrope Eigenschaften verfügt.

Ein weiteres Beispiel für Nanopartikel mit anisotropen spektralen Eigenschaften sind längliche Metall-Nanopartikel, bei welchen für Plasmonenschwingungen parallel und senkrecht zur leichten Achse zwei voneinander getrennte Resonanzfrequenzen auftreten. Abhängig von der Ausrichtung der langen Achse der Partikel zur Polarisation des einfallenden Lichtes werden diese beiden Resonanzfrequenzen anteilig angeregt. Durch die Messung der relativen Anteile dieser beiden Resonanzfrequenzen im Auslöschungs- oder Streuspektrum der Partikel ist somit eine Bestimmung der räumlichen Ausrichtung der langen Partikelachse relativ zur Polarisationsachse des einfallenden, auf die Partikel gerichteten, Lichtes möglich, siehe z.B. Pérez-Juste et al., Adv. Funct. Mater. 15 (2005) 1065.

### ii) Anisotrop polarisierende Nanopartikel:

Partikel mit einer Polarisationswirkung auf das gestreute, emittierte oder ausgelöschte Licht stellen eine weitere Variante für optisch anisotrope Nanopartikel dar. Möglich Beispiele sind polarisiert lumineszierende Partikel wie halbleitende Quantum Dots, Rods oder Wires. Insbesondere elongierte Partikel dieses Typs weisen ab einem Aspektverhältnis von 2:1 einen hohen Polarisationsgrad des emittierten Lichtes auf, siehe Hu et al., Science 292 (2001) 2060. Auch längliche metallische Nanopartikel, siehe Perez-Juste et al., Coordination Chemistry Review 249 (2005) 1870 oder in der Lösung verdünnte Mesomere von Flüssigkristallen können das einfallende Licht polarisieren.

Wird nun durch einen Polarisator oder Analysator im Strahlengang eine Vorzugsrichtung induziert, so kann über die Polarisationswirkung der Partikel auf ihre räumliche Ausrichtung geschlossen werden.

### 4. Optischer Aufbau:

Der optische Aufbau ist geeignet, das Maß der von den Nanopartikeln induzierten optischen Anisotropie zeitaufgelöst zu vermessen, um somit auf das Relaxationsverhalten der Partikel zu schließen. Der genaue Aufbau hängt von der Art der optischen Anisotropie ab, welche für die Messung herangezogen wird. Beispielhaft und ohne Beschränkung ist im Folgenden jeweils ein möglicher optischer Aufbau für die Messgrößen Spektrum und Polarisation aufgezeigt:

### i) Messgröße Spektrum: wird die räumliche Ausrichtung der Partikel in

Hierbei wird die räumliche Ausrichtung der Partikel in der Lösung über Veränderungen in ihrem Spektrum erfasst. Die verwendeten Partikel weisen hierbei ein anisotropes Auslöschungs-, Emissions- oder Streuspektrum auf, aus welchem auf die Ausrichtung der Anisotropieachse relativ zu einer durch mindestens einen Polarisator vorgegebenen Vorzugsrichtung geschlossen werden kann.

Als Beispiel sei hier ein möglicher Aufbau zur Messung des Relaxationsverhaltens von elongierten metallischen Nanopartikeln aufgeführt. Die Partikel sind dabei so beschaffen, dass in ihnen Plasmonen-Resonanzen auftreten, wobei die Resonanzfrequenz fₚₐᵣ für Plasmonenschwingungen parallel zur langen Achse spektral getrennt sei von der Resonanzfrequenz fₛₑₙₖ für Plasmonenschwingungen senkrecht zur langen Achse der Partikel. Des Weiteren sollen die Partikel so beschaffen sein, dass ihre lange Achse entlang eines äußeren Feldes ausgerichtet werden kann.

Die in einer sich in der Messzelle befindlichen Lösung dispergierten Nanopartikel werden mit linear polarisiertem Licht aus einer weißen Lichtquelle bestrahlt. Ein externer Stimulus sorgt für eine räumliche Ausrichtung der Partikel. Während einer zeitlichen Veränderung der Amplitude und/oder der Richtung des externen Stimulus bzw. Feldes wird das gestreute oder ausgelöschte Licht des Partikelensembles über geeignete Filter in zwei separaten Detektoren bei den beiden charakteristischen Plasmonenresonanzfrequenzen der Nanopartikel betrachtet. Aus dem aufgenommen zeitlichen Verlauf des Intensitätsverhältnisses aus beiden Detektoren bei einem bestimmten Programm zur Veränderung des externen Stimulus lässt sich sodann das Relaxationsverhalten der Nanopartikel bestimmen, aus welchem auf die mittlere Belegung der Partikel mit spezifisch an dieselben gebundenen Zielmolekülen geschlossen werden kann.

Dieses Messprinzip kann noch dahingehend variiert werden, dass zur Beleuchtung der Partikel unpolarisiertes Licht verwendet wird, und die Vorzugsrichtung erst durch einen Analysator im Strahlengang des gestreuten oder ausgelöschten Lichtes erzeugt wird. Auch eine geeignete Kombination mehrerer Polarisatoren und Analysatoren ist im Rahmen der vorliegenden Erfindung denkbar. Des Weiteren können auch andere Arten von Nanopartikeln mit spektral anisotroper Auslöschung, Emission oder Streuung verwendet werden.

### ii) Messgröße Polarisation:

Bei der Verwendung von licht-polarisierenden Nanopartikel kann die Polarisation des von den Partikeln gestreuten, emittierten oder ausgelöschten Lichtes zur Messung der mittleren räumlichen Ausrichtung des Partikelensembles herangezogen werden, um die Relaxation der Partikel bei einer Veränderung des externen Stimulus zu erfassen.

Im Rahmen eines besonders einfachen Messaufbaus wird die Messzelle mit unpolarisiertem Licht aus einer weißen Lichtquelle bestrahlt. Abhängig vom Grad der mittleren räumlichen Ausrichtung des Partikelensembles ergibt sich somit im gestreuten oder ausgelöschten Licht ein linearer Polarisationsanteil. Das aus der Probe austretende Licht wird nun über einen Analysator auf einen Detektor geführt, und es wird die zeitliche Veränderung des Intensitätssignals unter Veränderung der Amplitude und/oder Richtung des externen Stimulus aufgezeichnet. Hieraus lässt sich dann wieder das Relaxationsverhalten der Nanopartikel und somit die mittlere Belegung der Partikel mit spezifisch angebundenen Zielmolekülen bestimmen.

Alternativ kann an Stelle eines Analysators für das aus der Probe austretende Licht auch das einfallende Licht mittels Polarisator polarisiert werden, oder aber eine geeignete Kombination mehrerer Polarisatoren und Analysatoren verwendet werden.

### Beschreibung und Übersicht über die Figuren:

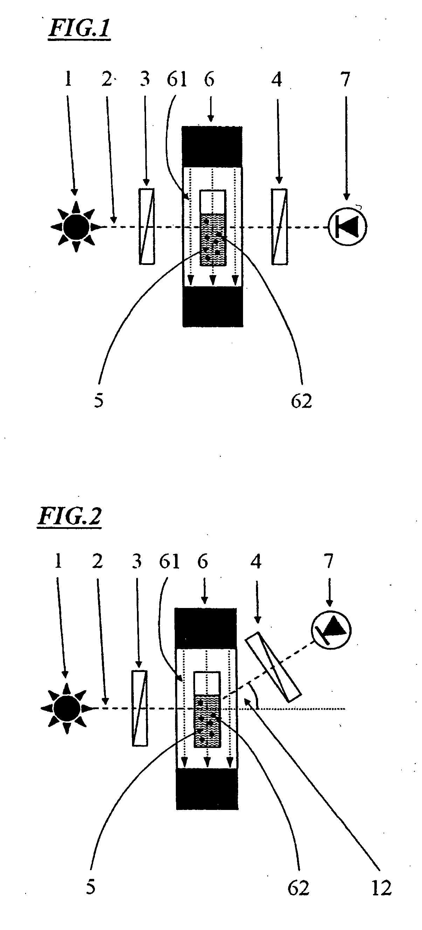

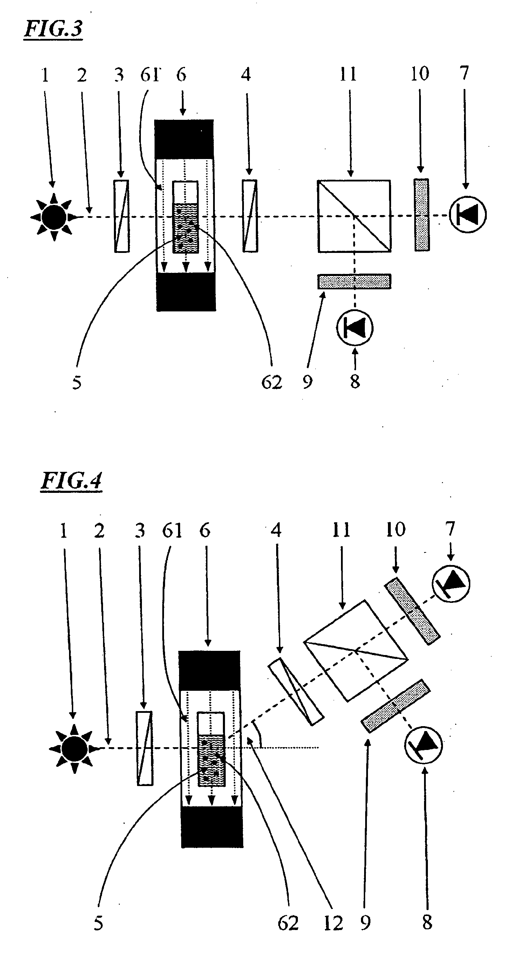

In den Zeichnungen ist der Erfindungsgegenstand beispielhaft dargestellt. Es zeigen die Fig. 1 und 2 mögliche Realisierungen des Messaufbaus für die Messgröße Polarisation und die Fig. 3 und 4 Realisierungen des Messaufbaus für die Messgröße Spektrum. Hierbei beziehen sich Fig. 1 und Fig. 3 auf Messungen der Auslöschung und Fig. 2 und Fig. 4 auf Messungen der Streuung oder Emission.

Allen möglichen Messaufbauten ist gemein, dass mit Hilfe einer beliebigen Lichtquelle 1 ein paralleler Lichtstrahl 2 erzeugt wird, welcher auf die Messzelle 5 gelenkt wird. Die Lichtquelle 1 kann hierbei sowohl monochromatisch, also z.B. ein Laser, als auch polychromatisch, also z.B. eine Glühwendel sein, welcher bzw. welche polarisiertes oder unpolarisiertes Licht emittiert.

Die Messzelle 5 enthält eine Lösung, welche neben Zusatzstoffen die zu analysierenden Ziel-Moleküle und die optisch anisotropen Nanopartikel 62 enthält. Die optischen Anisotropieachsen der Nanopartikel können durch einen externen Stimulus, also insbesondere durch ein Feld, ausgerichtet werden, welcher bzw. welches von einer geeigneten elektromagnetische Felder generierenden Apparatur 6 erzeugt wird und dessen Feld 61 hinsichtlich Amplitude und/oder Richtung verändert werden kann.

Mit Hilfe von mindestens einem Polarisator 3 und/oder Analysator 4 wird mindestens eine Vorzugsrichtung definiert, relativ zu welcher die von den Nanopartikeln induzierte optische Anisotropie im Detektor 7 in Abhängigkeit von der zeitlichen Veränderung des Stimulus bzw. Feldes vermessen wird.

Ist die Art der von den Nanopartikeln induzierten optischen Anisotropie eine Polarisationswirkung, so sind die oben beschriebenen Komponenten ausreichend für den optischen Aufbau.

Entsprechende Beispiele sind in Fig. 1 und Fig. 2 dargestellt, wobei Fig. 1 sich auf die Messung des von den Nanopartikeln ausgelöschten Lichtes bezieht. Fig. 2 dagegen zeigt einen möglichen Aufbau für Messungen der Streuung oder Emission, bei welchem der Analysatorstrahlengang um einen Raumwinkel 12 gegenüber dem einfallenden Lichtstrahl 2 gedreht ist.

Fig. 3 und Fig. 4 dagegen zeigen beispielhaft mögliche Messaufbauten für den Fall, dass die Nanopartikel spektral anisotrop Licht auslöschen, streuen oder emittieren. In diesem Fall werden mindestens zwei Wellenlängen ausgewählt, deren spektrale Intensität charakteristisch für die räumliche Ausrichtung der Nanopartikel ist. Im einfachsten Fall erfolgt die Analyse hierbei mit Hilfe eines Strahlteilers 11, welcher das von den Nanopartikeln ausgelöschte, gestreute oder emittierte Licht in mindestens zwei getrennte Strahlengänge aufteilt.

Anschließend können mit Hilfe von Farbfiltern 9,10 mindestens zwei charakteristische Spektralbereiche ausgewählt und in den Detektoren 7,8 vermessen werden. Das Verhältnis der mittels der mindestens zwei getrennten Detektoren gemessenen Lichtintensität erlaubt die Bestimmung der Ausrichtung der Nanopartikel, welche in Abhängigkeit von der zeitlichen Veränderung des auf dieselben einwirkenden Stimulus bzw. Feldes aufgenommen wird.

Fig. 3 bezieht sich auf die Messung des von den Nanopartikeln ausgelöschten Lichtes. Fig. 4 dagegen zeigt einen möglichen Aufbau zur Messungen der Streuung oder Emission, bei welchem der Analysatorstrahlengang um einen Raumwinkel 12 gegenüber dem einfallenden Lichtstrahl 2 gedreht ist.

## Patentansprüche

1. Verfahren zur molekularen Detektion anhand der Ermittlung bzw. Messung des Relaxationsverhaltens, insbesondere der Relaxationszeit, von in einem fluiden Medium dispergierten bzw. suspendierten Nanopartikeln, an welche bzw. an deren Oberfläche die Ziel-Moleküle, -Molekülsequenzen, -Molekülteile oder -Organismen spezifisch oder unspezifisch angebunden sind,
- wobei die genannten derart beaufschlagten Partikel in der Dispersion bzw. Suspension durch Einwirkung eines externen Stimulus, vorzugsweise eines gerichteten Feldes, insbesondere eines elektrischen und/oder magnetischen Feldes, zumindest überwiegend in einer Raum-Richtung ausrichtbar sind, und
- wobei die genannten Partikel in der Dispersion bzw. Suspension entweder von einem zuerst stochastischen, statistisch ungeordneten, ungerichteten Zustand ausgehend, in einen schließlich zumindest überwiegend einheitlich gerichteten Zustand übergeführt werden oder umgekehrt,
- und das Verhalten bzw. die Eigenschaften der Dispersion bzw. Suspension in jedem der beiden Zustände und/oder zwischen diesen Zuständen, und insbesondere die Zeit zwischen den beiden Zuständen, erfasst wird und aus dem so ermittelten RelaxationsVerhalten auf die Konzentration und/oder Größe und/oder Eigenschaft der an die Mikro- oder Nanopartikel gebundenen Ziel-Moleküle, -Molekülsequenzen, -Molekülteile oder - Organismen geschlossen wird,
- wobei für die Relaxationsverhaltens-Ermittlung eine Suspension bzw. Dispersion eingesetzt wird, deren Nanopartikel - neben ihrer geometrischen Lage-Ausrichtbarkeit - optische Anisotropie aufweisen,
- wobei in die genannte Suspension bzw. Dispersion entweder
a), insbesondere linear, polarisiertes oder
b) nicht polarisiertes
Licht eingestrahlt wird, und
- wobei das aus der Suspension oder Dispersion
- entweder direkt in Einstrahl-Richtung in bzw. durch Absorption aufgenommene oder im Winkel zu derselben als Streulicht austretende Licht als solches direkt, oder nach Durchgang durch ein der vom Licht durchquerten Dispersion bzw. Suspension nachgeschaltetes Polarisationsfilter einer optometrischen Analyse unterworfen wird,
**dadurch gekennzeichnet,**
**dass** die in der Dispersion bzw. Suspension vorliegenden durch den externen Stimulus, insbesondere durch das externe Feld, ausrichtbaren Nanopartikel Nanopartikel aus oder mit Edelmetallen sind, in welchen durch einfallendes Licht anisotrope Plasmonenresonanzen mit charakteristischen Streu- und/oder Auslöschungsspektren oder Änderungen in denselben angeregt werden.

2. Verfahren nach Anspruch 1, **dadurch gekennzeichnet, dass** bei der optometrischen Analyse als Messgröße das optische Spektrum des von den Mikro- oder Nanopartikeln in der Dispersion bzw. Suspension gestreuten, oder ausgelöschten Lichtes, und insbesondere die Intensität bzw. die Änderung der Intensität desselben, herangezogen wird.

3. Verfahren nach einem der vorangehenden Ansprüche, **dadurch gekennzeichnet, dass** der bzw. das auf die mit dem/der an sie anbindenden Molekül, Molekülsequenz, Molekülteil bzw. Organismus beaufschlagten Mikro- oder Nanopartikel wirkende Stimulus bzw. Feld im Laufe der Messung in seiner Amplitude und/oder Richtung verändert wird und die Relaxation der genannten Partikel hin zu der durch den bzw. das neue(n) veränderte(n) Stimulus bzw. Feld gegebenen Gleichgewichtsposition zeit-, frequenz- oder phasenabhängig ermittelt und aufgezeichnet wird.

4. Verfahren nach Anspruch 3, **dadurch gekennzeichnet, dass** der bzw. das auf die mit den Ziel-Molekülen beladenen Nanopartikel in der Dispersion bzw. Suspension einwirkende Stimulus bzw. Feld diskret auf einem bestimmten Wert gehalten oder aber periodisch verändert wird.

5. Verfahren nach einem der vorangehenden Ansprüche, **dadurch gekennzeichnet, dass** in der Dispersion bzw. Suspension als durch ein externes Magnetfeld ausrichtbare, optisch anisotrope magnetisierbare Nanopartikel oder von vornherein selbst ein magnetisches Moment aufweisende Partikel oder mit magnetischen Komponenten versehene, Magnet-Komposit-Nanopartikel darstellende, mit Kern-Hülle-Struktur, oder ganz oder teilweise mit magnetischem Material gefüllte Carbon-Nanotubes, oder längliche bzw. elongierte Partikel, wie z.B. Nanotubes, mit magnetischen Materialien an zumindest einem von deren Polen bzw. Enden, eingesetzt werden.

6. Verfahren nach einem der vorangehenden Ansprüche, **dadurch gekennzeichnet, dass** in der Dispersion bzw. Suspension als durch einen externen Stimulus, insbesondere durch ein externes Feld, ausrichtbare, optisch anisotrope Nanopartikel mit kristalliner oder amorpher sphärischer oder elongierter Form, welche in zumindest einer der drei Dimensionen jeweils eine Größenausdehnung von 1 bis 1000 nm, insbesondere von 2 bis 100 nm, aufweisen, eingesetzt werden.

7. Verfahren nach einem der vorangehenden Ansprüche, **dadurch gekennzeichnet, dass** die an die optisch anisotropen Nanopartikel in der Dispersion bzw. Suspension bzw. an deren Oberfläche gebundenen Ziel-Moleküle, -Molekülsequenzen, -Molekülteile oder - Organismen aus der Gruppe Viren, Bakterien, Nukleinsäuresequenzen, Antikörper, Antigene, Proteine, Moleküle und chemischer Verbindungen, insbesondere Oligomer- oder Polymer-Moleküle, ausgewählt sind.

8. Verfahren nach einem der vorangehenden Ansprüche, **dadurch gekennzeichnet, dass** für die Dispersion bzw. Suspension für die anisotropen bzw. anisotropisierten Nanopartikel als fluides Dispersionsmedium eine Flüssigkeit eingesetzt wird, welche zumindest in jenem Wellenlängenbereich, welcher zur Ermittlung der optischen Anisotropie der in derselben verteilten Mikro- oder Nanopartikel dient, zumindest optisch teiltransparent ist.

9. Vorrichtung zur Durchführung des Verfahren nach einem der vorangehenden Ansprüche, welche eine - einen, vorzugsweise parallelen, Strahl (2) nicht polarisierten, oder gleich von vornherein polarisierten Lichts - abstrahlende Lichtquelle (1), vorzugsweise Laser-Lichtquelle, gegebenenfalls eine von demselben zu durchsetzende Polarisations-Einrichtung (3), insbesondere ein Polarisationsfilter bzw. einen Polarisator, ein licht-transparentes Gefäß, insbesondere eine derartige Küvette (5), mit der mit einem fluiden Medium zubereiteten Dispersion bzw. Suspension von optisch anisotropen Nanopartikeln, an welche bzw. an deren Oberfläche spezifisch anbindbare Ziel-Moleküle oder -Organismen angebunden sind, eine einen zur einheitlichen Ausrichtung der Nanopartikel befähigten Stimulus, insbesondere einen ein derartiges magnetisches und/oder elektrisches Feld in der Partikel-Dispersion bzw. -Suspension generierenden Stimulus, aussendende insbesondere hinsichtlich Amplitude und/oder Richtung steuerbare, Feld(61)-Abstrahleinrichtung (6), eine, dem die Partikel-Suspension enthaltenden Gefäß, insbesondere der Küvette (5), nachgeordnete, gegebenenfalls in Lichtstrahlrichtung oder in einem Winkel (α) zu derselben angeordnete Polarisations-Einrichtung (4), insbesondere Polarisationsfilter bzw. Analysator, und eine die Veränderung der Polarisationsrichtung und/oder Intensität des austretenden Lichtes in Folge der durch den genannten Stimulus, insbesondere durch das Feld (61) generierten Ausrichtung bzw. Nichtausrichtung der Mikro- oder Nanopartikel (62) ermittelnde und registrierende Detektions-Einrichtung (7) umfasst,
**dadurch gekennzeichnet,**
**dass die in der Dispersion bzw. Suspension vorliegenden durch den externen Stimulus, insbesondere durch das externe Feld, ausrichtbaren Nanopartikel Nanopartikel aus oder mit Edelmetallen sind, in welchen einfallendes Licht anistrope Plasmonenresonanzen mit charakteristischen Streu- und/oder Auslöschungsspektren oder Änderungen in denselben anregt.**

## Claims

1. A method for the molecular detection based on the determination, or measurement, respectively, of the relaxation behavior, in particular of the relaxation time, of nanoparticles dispersed, or suspended in a fluid medium, to which, or on the surface of which, the target molecules, target molecule sequences, target molecule particles, or target organisms are bound specifically or non-specifically,
- wherein said particles charged in this manner may be aligned, at least predominantly, in the dispersion or suspension in a spatial direction under the influence of an external stimulus, preferably a directional field, in particular an electric and/or magnetic field, and
- wherein said particles are transferred in the dispersion or suspension, either based from an initially stochastic, statically inordinate, non-directional state, to a finally at least predominantly uniformly directional state, or vice versa,
- and the behavior, or the properties of the dispersion or suspension is detected in each of the two states, and/or between said states, and in particular the time between the two states is detected, and a conclusion is drawn from the relaxation behavior determined in this manner as to the concentration, and/or size, and/or property of the target molecules, target molecule sequences, target molecule particles, or target organisms bound to the micro or nanoparticles,
- wherein, for determining the relaxation behavior, a suspension or dispersion is utilized, the nanoparticles of which - in addition to its capability of geometric location alignment - have an optical anisotropy,
- wherein either
a) particularly linear, polarized, or
b) non-polarized
light is radiated into said suspension or dispersion, and
- wherein the light exiting as scattered light
- either directly absorbed in the irradiation direction, or by means of absorption, or at an angle to the same, as such is subjected to an optometric analysis, directly, or after passing through a polarization filter downstream of the dispersion or suspension being traversed by the light,
**characterized in that**
the nanoparticles present in the dispersion or suspension, capable of being aligned by the external stimulus, in particular by means of the external field, are comprised, or contain precious metals, in which anisotropic Plasmon resonances having characteristic scattering and/or extinguishing spectra, or changes in the same, are excited by incident light.

2. The method according to claim 1, **characterized in that** with the optometric analysis the optical spectrum of the light scattered or extinguished by the micro or nanoparticles in the dispersion or suspension, and in particular the intensity, or the change of intensity of the same, is utilized.

3. The method according to one of the preceding claims, **characterized in that** the stimulus, or field, respectively, acting upon the micro or nanoparticles with the molecule, molecule sequence, molecule particle, or molecule organism binding on the same, is changed during the course of the measurement in terms of its amplitude, and/or direction, and the relaxation of said particles towards the equilibrium position given by the newly changed stimulus, or field is determined and recorded, depending on the time, frequency, or phase.

4. The method according to claim 3, **characterized in that** the stimulus, or field, respectively, acting upon the nanoparticles charged with the target molecules in the dispersion or suspension, is discretely maintained at a certain value, or is periodically changed.

5. The method according to one of the preceding claims, **characterized in that** carbon nanotubes, representing magnetic composite nanoparticles, with a core/sheath structure, or completely or partially filled with magnetic material, are utilized in the dispersion or suspension, on at least one of the poles or ends thereof, as optical, anisotropic magnetizable nanoparticles capable of being aligned by means of an external magnetic field, or as particles having themselves a magnetic moment from the onset, or having magnetic components, or long, or elongated particles, such as nanotubes, having magnetic materials.

6. The method according to one of the preceding claims, **characterized in that** optical, anisotropic nanoparticles with crystalline, or amorphous, spherical or elongated shape, capable of being aligned by means of an external stimulus, in particular by means of an external field, are utilized in the dispersion or suspension, which each have a size dimension of 1 to 1000 nm, in particular 2 to 100 nm, at least in one of the three dimensions.

7. The method according to one of the preceding claims, **characterized in that** the target molecules, target molecule sequences, target molecule particles, or target organisms bound to the optical, anisotropic nanoparticles, or to the surfaces thereof, respectively, in the dispersion or suspension are selected from the group of viruses, bacteria, nucleic acid sequences, antibodies, antigens, proteins, molecules, and chemical compounds, in particular from oligomer, or polymer molecules.

8. The method according to one of the preceding claims, **characterized in that** a liquid is utilized as the fluid dispersion medium for the dispersion or suspension for the anisotropic, or anistotropized nanoparticles, respectively, which is at least optically partially transparent, at least **in that** wave length area, which serves for determining the optical anisotropy of the micro or nanoparticles distributed in the same.

9. A device for carrying out the method according to one of the preceding claims, comprising a light source (1) - radiating a preferably parallel beam (2) of a non-polarized, or polarized light from the onset - preferably a laser light source, optionally a polarization unit (3) to be interspersed by the same, in particular a polarization filter, or a polarizer, a light-transparent vessel, in particular such a cuvette (5), having the dispersion or suspension of optical anisotropic nanoparticles prepared with a fluid medium, to which, or at the surface of which specific binding target molecules or target organisms are bound, a field (61) jet blasting unit (6) sending a stimulus capable of a uniform alignment of the nanoparticles, in particular a stimulus generating such a magnetic and/or electric field in the particle dispersion or suspension, which is controllable, in particular with regard to the amplitude and/or direction, a polarization unit (4) arranged downstream of the vessel containing the particle suspension, in particular the cuvette (5), optionally arranged in the direction of the light beam, or at an angle (α) toward the same, in particular a polarization filter, or analyzer, and a detection unit (7) detecting and registering the change in polarization direction, and/or intensity of the light exiting as a result of said stimulus, in particular the alignment or non-alignment of the microparticles or nanoparticles (62) generated by the field (61),
**characterized in that**
the nanoparticles present in the dispersion or suspension, which may be aligned by means of the external stimulus, in particular by means of the external field, are comprised of, or contain precious metals, in which incidental light excites anisotropic Plasmon resonances having characteristic scattering and/or extinguishing spectra, or changes in the same.

## Revendications

1. Procédé de détection moléculaire par détermination ou mesure du comportement de relaxation, notamment du temps de relaxation, de nanoparticules dispersées ou suspendues dans un milieu fluide, auxquelles ou à la surface desquelles les molécules, séquences de molécules, parties de molécules ou organismes cibles sont reliés de manière spécifique ou non spécifique,
- lesdites particules ainsi exposées étant orientables au moins essentiellement dans une direction de l'espace dans la dispersion ou suspension par l'action d'un stimulus externe, de préférence d'un champ orienté, notamment d'un champ électrique et/ou magnésique, et
- lesdites particules étant transformées dans la dispersion ou suspension d'un état initial stochastique, statistiquement désordonné, non orienté, en un état final au moins essentiellement orienté uniformément, ou l'inverse,
- et le comportement ou les propriétés de la dispersion ou suspension dans chacun des deux états et/ou entre ces états, et notamment le temps entre les deux états, étant déterminés, et la concentration et/ou la taille et/ou les propriétés des molécules, séquences de molécules, parties de molécules ou organismes cibles reliés aux micro- ou nanoparticules étant déterminées à partir du comportement de relaxation ainsi déterminé,
- une suspension ou dispersion dont les nanoparticules présentent une anisotropie optique, en plus de leur orientabilité géométrique, étant utilisée pour la détermination du comportement de relaxation,
- ladite suspension ou dispersion étant exposée à une lumière
a) notamment linéaire, polarisée ou
b) non polarisée
et
- la lumière provenant de la suspension ou dispersion,
- soit absorbée directement dans la direction incidente en ou par absorption, soit sortant sous forme de lumière diffusée à un angle de celle-ci, étant soumise à une analyse optométrique en tant que telle directement ou après passage au travers d'un filtre de polarisation disposé en aval de la dispersion ou suspension traversée par la lumière,
**caractérisé en ce que**
les nanoparticules orientables par le stimulus externe, notamment par le champ externe, présentes dans la dispersion ou suspension, sont des nanoparticules en ou comprenant des métaux nobles, dans lesquelles des résonances plasmoniques anisotropes présentant des spectres de diffusion et/ou d'extinction caractéristiques ou des modifications de ceux-ci sont excitées par la lumière incidente.

2. Procédé selon la revendication 1, **caractérisé en ce que** le spectre optique de la lumière diffusée par les micro- ou nanoparticules dans la dispersion ou suspension ou éteinte, et notamment l'intensité ou la modification de l'intensité de celle-ci, est utilisé en tant que grandeur de mesure lors de l'analyse optométrique.

3. Procédé selon l'une quelconque des revendications précédentes, **caractérisé en ce que** le stimulus ou champ agissant sur les micro- ou nanoparticules exposées auxquelles la molécule, séquence de molécule, partie de molécule ou organisme est relié est modifié au cours de la mesure au niveau de son amplitude et/ou de sa direction, et la relaxation desdites particules est déterminée et enregistrée en fonction du temps, de la fréquence ou de la phase jusqu'à la position d'équilibre donnée par le nouveau stimulus ou champ modifié.

4. Procédé selon la revendication 3, **caractérisé en ce que** le stimulus ou champ agissant sur les nanoparticules chargées avec les molécules cibles dans la dispersion ou suspension est maintenu discrètement à une valeur déterminée ou modifié périodiquement.

5. Procédé selon l'une quelconque des revendications précédentes, **caractérisé en ce que** des nanoparticules magnétisables, optiquement anisotropes, orientables par un champ magnétique externe, ou des particules présentant d'entrée elles-mêmes un moment magnétique, ou des nanotubes de carbone munis de composants magnétiques, formant des nanoparticules composites magnétiques, à structure moyen-enveloppe, ou remplis en totalité ou en partie avec un matériau magnétique, ou des particules longitudinales ou allongées, telles que p. ex. des nanotubes, comprenant des matériaux magnétiques à au moins un de leurs pôles ou extrémités, sont utilisés dans la dispersion ou suspension.

6. Procédé selon l'une quelconque des revendications précédentes, **caractérisé en ce que** des nanoparticules optiquement anisotropes, orientables par un stimulus externe, notamment par un champ externe, de forme sphérique ou allongée cristalline ou amorphe, qui présentent dans au moins une des trois dimensions à chaque fois une taille de 1 à 1 000 nm, notamment de 2 à 100 nm, sont utilisées dans la dispersion ou suspension.

7. Procédé selon l'une quelconque des revendications précédentes, **caractérisé en ce que** les molécules, séquences de molécules, parties de molécules ou organismes cibles reliés aux nanoparticules optiquement anisotropes dans la dispersion ou suspension ou à leur surface sont choisis dans le groupe constitué par les virus, les bactéries, les séquences d'acides nucléiques, les anticorps, les antigènes, les protéines, les molécules et les composés chimiques, notamment les molécules oligomères ou polymères.

8. Procédé selon l'une quelconque des revendications précédentes, **caractérisé en ce qu'**un liquide qui est au moins partiellement transparent optiquement au moins dans la plage de longueurs d'onde utilisée pour la détermination de l'anisotropie optique des micro- ou nanoparticules dispersées dans celui-ci est utilisé en tant que milieu de dispersion fluide pour la dispersion ou suspension pour les nanoparticules anisotropes ou anisotropisées.

9. Dispositif pour la réalisation du procédé selon l'une quelconque des revendications précédentes, qui comprend une source de lumière (1) émettant un faisceau (2), de préférence parallèle, de lumière non polarisée ou polarisée d'entrée, de préférence une source de lumière laser, éventuellement un dispositif de polarisation (3) à traverser par celle-ci, notamment un filtre de polarisation ou un polarisateur, un récipient transparent à la lumière, notamment une cuvette (5) comprenant la dispersion ou suspension préparée avec un milieu fluide de nanoparticules optiquement anisotropes, auxquelles ou à la surface desquelles des molécules ou organismes cibles reliables de manière spécifique sont reliés, un dispositif (6) d'émission d'un champ (61) émettant un stimulus permettant l'orientation uniforme des nanoparticules, notamment un stimulus générant un champ magnétique et/ou électrique dans la dispersion ou suspension de particules, ajustable notamment au regard de l'amplitude et/ou de la direction, un dispositif de polarisation (4) agencé après le récipient contenant la suspension de particules, notamment la cuvette (5), éventuellement dans la direction du faisceau de lumière ou à un angle (α) par rapport à celle-ci, notamment un filtre de polarisation ou un analyseur, et un dispositif de détection (7) déterminant et enregistrant la modification de la direction de polarisation et/ou d'intensité de la lumière sortante en conséquence de l'orientation ou de la non-orientation des micro- ou nanoparticules (62) générée par ledit stimulus, notamment par le champ (61), **caractérisé en ce que**
les nanoparticules orientables par le stimulus externe, notamment par le champ externe, présentes dans la dispersion ou suspension, sont des nanoparticules en ou comprenant des métaux nobles, dans lesquelles la lumière incidente excite des résonances plasmoniques anisotropes présentant des spectres de diffusion et/ou d'extinction caractéristiques ou des modifications de ceux-ci.
